# EUROPÄISCHE PATENTSCHRIFT

(11) **EP 1 244 454 B1**
(45) Veröffentlichungstag und Bekanntmachung des Hinweises auf die Patenterteilung: **26.04.2006**
(21) Anmeldenummer: 00966165.3
(22) Anmeldetag: 14.10.2000
(51) Int. Cl.: A61K 31/517

(54) **VERWENDUNG VON 2-AMINO-3,4-DIHYDRO-CHINAZOLINEN ZUR HERSTELLUNG EINES MEDIKAMENTS ZUR BEHANDLUNG ODER PROPHYLAXE VON ISCHÄMISCHEN ZUSTÄNDEN**
USE OF 2-AMINO-3,4-DIHYDRO-QUINAZOLINES FOR PRODUCING A MEDICAMENT FOR TREATING OR PREVENTING DISEASES CAUSED BY ISCHAEMIC CONDITIONS
UTILISATION DE 2-AMINO-3,4-DIHYDRO-QUINAZOLINES POUR PREPARER UN MEDICAMENT UTILISE POUR ASSURER LE TRAITEMENT OU LA PROPHYLAXIE D'AFFECTIONS INDUITES PAR DES ETATS ISCHEMIQUES

(30) Priorität: 27.10.1999 DE 19951702
(43) Veröffentlichungstag der Anmeldung: 02.10.2002
(73) Patentinhaber: Sanofi-Aventis Deutschland GmbH, 65929 Frankfurt am Main (DE)
(72) Erfinder: WEICHERT, Andreas, 22391 Hamburg (DE); ALBUS, Udo, 61197 Florstadt (DE); JANSEN, Hans-Willi, 65527 Niedernhausen (DE)
(86) Internationale Anmeldenummer: PCT/EP2000/010127
(87) Internationale Veröffentlichungsnummer: WO 2001/030328

(56) Entgegenhaltungen:
- EP-A- 0 530 994
- EP-A- 0 664 128
- EP-A- 0 869 116
- BENOS D J ET AL: "ENVELOPE GLYCOPROTEIN GP120 OF HUMAN IMMUNODEFICIENCY VIRUS TYPE 1 ALTERS ION TRANSPORT IN ASTROCYTES: IMPLICATIONS FOR AIDS DEMENTIA COMPLEX" PROCEEDINGS OF THE NATIONAL ACADEMY OF SCIENCES OF USA, NATIONAL ACADEMY OF SCIENCE. WASHINGTON, US, Bd. 91, Januar 1994 (1994-01), Seiten 494-498, XP001015881 ISSN: 0027-8424
- BENOS DALE J ET AL: "Cytokines and HIV envelope glycoprotein gp120 stimulate Na+/H+ exchange in astrocytes." JOURNAL OF BIOLOGICAL CHEMISTRY, Bd. 269, Nr. 19, 1994, Seiten 13811-13816, XP001038417 ISSN: 0021-9258
- SCHOLZ W ET AL: "EFFECTS OF NA+/H+ EXCHANGE INHIBITORS IN CARDIAC ISCHEMIA" JOURNAL OF MOLECULAR AND CELLULAR CARDIOLOGY, XX, XX, Bd. 24, Nr. 7, 1. Juli 1992 (1992-07-01), Seiten 733-739, XP002048402 ISSN: 0022-2828
- AVKIRAN M: "Rational basis for use of sodium-hydrogen exchange inhibitors in myocardial ischemia" AMERICAN JOURNAL OF CARDIOLOGY, CAHNERS PUBLISHING CO., NEWTON, MA,, US, Bd. 83, Nr. 10A, 20. Mai 1999 (1999-05-20), Seiten 10G-18G, XP002130273 ISSN: 0002-9149
- MYERS M L ET AL: "HYDROGEN PEROXIDE INDUCED IMPAIRMENT OF POST-ISCHEMIC VENTRICULAR FUNCTION IS PREVENTED BY THE SODIUM-HYDROGEN EXCHANGE INHIBITOR HOE 642 (CARIPORIDE)" CARDIOVASCULAR RESEARCH, XX, XX, Bd. 40, Nr. 2, 1998, Seiten 290-296, XP000920530 ISSN: 0008-6363
- KARMAZYN M: "THE MYOCARDIAL SODIUM-HYDROGEN EXCHANGER (NHE) AND ITS ROLE IN MEDIATING ISCHEMIC AND REPERFUSION INJURY" KEIO JOURNAL OF MEDICINE, TOKYO, JP, Bd. 47, Nr. 2, 1998, Seiten 65-72, XP000920589 ISSN: 0022-9717
- LEVITSKY J ET AL: "SODIUM ION/HYDROGEN ION EXCHANGE INHIBITION: A NEW PHARMACOLOGIC APPROACH TO MYOCARDIAL ISCHEMIA AND REPERFUSION INJURY" JOURNAL OF CLINICAL PHARMACOLOGY, LIPPINCOTT CO, HAGERSTOWN, MD, US, Bd. 38, Nr. 10, 1998, Seiten 887-897, XP000920868 ISSN: 0091-2700

## Beschreibung

Verwendung von 2-Amino-3,4-dihydro-chinazolinen zur Herstellung eines Medikaments zur Behandlung oder Prophylaxe von durch ischämischen Zuständen bewirkten Krankheiten

Die Erfindung betrifft die Verwendung von 2-Amino-3,4-dihydro-chinazoline der Formel I 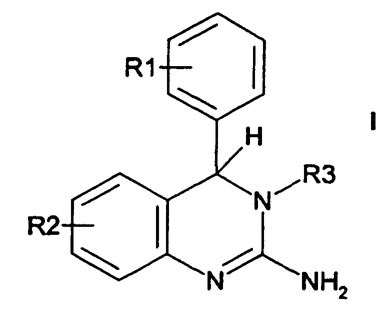 sowie von deren pharmazeutisch verträglichen Salzen zur Herstellung eines Medikaments zur Therapie und Prophylaxe ischämischer Zustände. In Formel I bedeuten:
- R1 und R2: Wasserstoff, F, Cl, Br, I, geradkettiges oder verzweigtes C₁-C₄- Alkyl, C₁₋C₄- Alkoxy,
- R3: Wasserstoff, geradkettiges oder verzweigtes C₁-C₄- Alkyl oder Phenyl,
wobei der Phenylkern unsubstituiert ist oder einen bis drei Substituenten ausgewählt aus der Gruppe bestehend aus F, Cl, CH₃ und CH₃O trägt.

Bevorzugt ist die Verwendung von Verbindungen der Formel I sowie von deren Salzen, in der bedeuten:
- R1 und R2: Wasserstoff, Fluor, Chlor, geradkettiges oder verzweigtes C₁-C₄- Alkyl, C₁₋C₄- Alkoxy;
- R3: Wasserstoff oder Methyl.

Enthält einer der drei Substituenten R1, R2 und R3 ein Asymmetriezentrum, so gehören sowohl S als auch R konfigurierte Verbindungen zur Erfindung. Die Verbindungen können als optische Isomere, als Diastereoisomere, als Racemate oder als Gemische derselben vorliegen.

Verbindungen der Formel I sind bekannt aus
EP 530 994, wo sie als Inhibitoren der HIV-Reversen Transkriptase in der Indikation AIDS beschrieben werden;
der US- Patentschrift 3 560 050, in welcher deren analgetische, diuretische und antiinflammatorische Wirkung beschrieben wird;
und aus Kosasayama et al., Chem. Pharm. Bull. 27, 880 (1979) und Ishikawa et al. Ibid, 28, 1357 (1980), wo für 2-Amino-4-phenyl-3,4-dihydrochinazolin eine Inhibierung der Thrombozytenaggregation gezeigt werden konnte.

Überraschenderweise wurde nun gefunden, dass sich diese bekannten Verbindungen durch eine Inhibition des Na⁺/H⁺-Austauschs auszeichnen. Somit sind sie infolge ihrer pharmakologischen Eigenschaften zur Herstellung von antiarrhythmischen Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindem. Wegen ihrer schützenden Wirkungen gegen pathologische hypoxische und ischämische Situationen können die erfindungsgemäß verwendeten Verbindungen der Formel I infolge Inhibition des zellulären Na⁺/H⁺⁻Austauschmechanismus als Arzneimittel zur Behandlung aller akuten oder chronischen durch Ischämie ausgelösten Schäden oder dadurch primär oder sekundär induzierten Krankheiten dienen. Dies betrifft ihre Verwendung als Arzneimittel für operative Eingriffe, z. B. bei Organ-Transplantationen, wobei die erfindungsgemäß verwendeten Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die erfindungsgemäß verwendeten Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des Zentralnervensystems, geeignet, wobei sie z. B. zur Behandlung des Schlaganfalls oder des Hirnödems geeignet sind. Darüber hinaus eignen sich die Verbindungen der Formel 1 ebenfalls zur Behandlungen von Formen des Schocks, wie beispielweise des allergischen, cardiogenen, hypovolämischen und des bakteriellen Schocks.

Darüber hinaus zeichnen sich die erfindungsgemäß verwendeten Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die erfindungsgemäß verwendeten Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien undhyperplasien, insbesondere bei Prostatahyperplasie bzw. Prostatahypertrophie verwendet werden.

Die erfindungsgemäß verwendeten Verbindungen sind wirkungsvolle Inhibitoren des zellulären Natrium-Protonen-Antiporters (Na⁺/H⁺ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die erfindungsgemäß verwendeten Verbindungen der Formel I für die präventive Therapie zur Verhinderung der Genese des Bluthochdrucks, beispielweise der essentiellen Hypertonie, geeignet.

Es wurde außerdem gefunden, daß erfindungsgemäß verwendeten Verbindungen der Formel I eine günstige Beeinflussung der Serumlipoproteine zeigen. Es ist allgemein anerkannt, daß für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter Serum-Lipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine (VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondem insbesondere die VLDL und LDL-Serumcholesterinfraktionen zu senken. Es wurde nun gefunden, daß die erfindungsgemäß verwendeten Verbindungen der Formel I bezüglich der Beeinflussung der Serumlipidspiegel wertvolle therapeutisch verwertbare Eigenschaften zeigen. So emiedrigen sie signifikant die erhöhte Serum Konzentration von LDL und VLDL, wie sie beispielweise durch erhöhte dietätische Einnahme einer cholesterin- und lipidreichen Kost oder bei pathologischen Stoffwechselveränderungen, beispielsweise genetisch bedingten Hyperüpidämien zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z. B. beim Diabetes vorkommen. Darüber hinaus führen die erfindungsgemäß verwendeten Verbindungen der Formel I zu einer deutlichen Reduktion der durch Stoffwechselanomalien induzierten Infarkte und insbesondere zu einer signifikanten Verminderung der induzierten Infarktgröße und dessen Schweregrades. Weiterhin führen die erfindungsgemäß verwendeten Verbindungen der Formel I zu einem wirksamen Schutz gegen durch Stoffwechselanomalien induzierte Endothelschädigungen. Mit diesem Schutz der Gefäße gegen das Syndrom der endothelialen Dysfunktion sind Verbindungen der Formel I wertvolle Arzneimittel zur Prävention und zur Behandlung koronarer Gefäßspasmen, der Atherogenese und der Atherosklerose, der linksventrikulären Hypertrophie und der dilatierten Kardiomyopathie, und thrombotischer Erkrankungen.

Die erfindungsgemäß verwendeten Verbindungen I finden deshalb vorteilhaft Verwendung
zur Herstellung eines Medikaments zur Behandlung von Hypercholesterinämie;
zur Herstellung eines Medikaments zur Prävention der Atherogenese;
zur Herstellung eines Medikaments zur Prävention und Behandlung der Atherosklerose;
zur Herstellung eines Medikaments zur Prävention und Behandlung von Krankheiten, die durch erhöhte Cholesterinspiegel ausgelöst werden;
zur Herstellung eines Medikaments zur Prävention und Behandlung von Krankheiten, die durch endotheliale Dysfunktion ausgelöst werden;
zur Herstellung eines Medikaments zur Prävention und Behandlung von atherosklerose-induzierter Hypertonie;
zur Herstellung eines Medikaments zur Prävention und Behandlung von atherosklerose-induzierter Thrombosen;
zur Herstellung eines Medikaments zur Prävention und Behandlung von Hypercholesterinämie- und endothelialer Dysfunktion induzierter ischämischer Schäden und postischämischer Reperfusionsschäden;
zur Herstellung eines Medikaments zur Prävention und Behandlung von Hypercholesterinämie- und endothelialer Dysfunktion induzierter cardialer Hypertrophien und Cardiomyopathien;
zur Herstellung eines Medikaments zur Prävention und Behandlung von Hypercholesterinämie- und endothelialer Dysfunktion induzierter koronarer Gefäßspasmen und myocardialer Infarkte;
zur Herstellung eines Medikaments zur Behandlung der genannten Leiden in Kombinationen mit blutdrucksenkenden Stoffen, bevorzugt mit Angiotensin Converting Enzyme (ACE)-Hemmem und Angiotensin-Rezeptorantagonisten. Eine Kombination eines NHE-Inhibitors der Formel I mit einem blutfettspiegelsenkenden Wirkstoff, bevorzugt mit einem HMG-CoA-Reduktaseinhibitor (z.B. Lovastatin oder Pravastatin), wobei letzterer eine hypolipidämische Wirkung herbeiführt und dadurch die hypolipidämischen Eigenschaften des NHE-Inhibitors der Formel I steigert, erweist sich als günstige Kombination mit verstärkter Wirkung und vermindertem Wirkstoffeinsatz.

Beansprucht wird die Verwendung der Natrium-Protonen-Austausch-Hemmem der Formel I zur Herstellung neuartiger Arzneimittel zur Senkung erhöhter Blutfettspiegel, sowie die Kombination von Natrium-Protonen-Austausch-Hemmem mit blutdrucksenkenden und/oder hypolipidämisch wirkenden Arzneimitteln.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnem, Suppositorien-Grundlagen, Tablettenhilfsstoffen, und anderen Wirkstoffträgem können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die erfindungsgemäß verwendeten aktiven Verbindungen I mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können
z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die erfindungsgemäß verwendeten aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierungen für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten

Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Experimenteller Teil

### Liste der Abkürzungen:

- DMSO: Dimethylsulfoxid
- RT: Raumtemperatur
- EE: Ethylacetat (EtOAc)
- Smp: Schmelzpunkt
- eq.: Äquivalent

### Beispiel 1: 2-Amino-4-(4-methoxy-phenyl)-3,4-dihydro-chinazolin-Hydrochlorid farbloser Fest-stoff, Smp. 169°C, M⁺+H= 254.

### Syntheseweg:

2-Amino-phenyl-(4-methoxy-phenyl)-keton wird mit 1.2 eq Formamidin-hydrochlorid in Gegenwart von Kaliumkarbonat unter Erhitzen in DMSO zum entsprechenden Chinazolin umgesetzt. Nach wäßriger Aufarbeitung wird das Rohprodukt mit 10%Pd/C in Ethanol hydriert, anschließende Säulenchromatographie und Hydrochloridbildung mit etherischem Chlorwasserstoff ergibt das Produkt als Feststoff.

Beispiel 2: 2-Amino-4-(3,4-dimethoxy-phenyl)-3,4-dihydro-chinazolinium-Fumarat, farbloser Feststoff, Smp. 179°C, M⁺+H= 284.

Die Synthese erfolgt analog Beispiel 1 unter Verwendung von 2-Amino-phenyl-(3,4-dimethoxy-phenyl)-keton. Zur Salzbildung wird die Base mit 1 eq Fumarsäure in EE umgesetzt.

### Beispiel 3: 2-Amino-4-(3,4-dimethoxy-phenyl)-3-methyl-3,4-dihydro-chinazolinium-Fumarat

farbloser Feststoff, Smp. 202°C, M⁺+H= 298.

### Syntheseweg:

Ausgehend von 2-Amino-phenyl-(3,4-dimethoxy-phenyl)-keton wird mit Methylamin/Titantetrachlorid und nachfolgender Reduktion mit Natriumborhydrid das entsprechende Benzhydrylamin hergestellt. Das Rohprodukt wird mit einem Überschuß an Bromcyan zum 3,4-Dihydro-chinazolin cyclisiert und säulenchromatographiert. Anschließende Salzbildung erfolgt wie unter Beispiel 2) beschrieben.

### Pharmakologische Daten:

### Inhibition des Na⁺/H⁺-Exchangers von Kaninchenerythrocyten

Weiße Neuseeland-Kaninchen (Ivanovas) erhielten eine Standard-Diät mit 2% Cholesterin für sechs Wochen, um den Na⁺/H⁺-Austausch zu aktivieren und so den Na⁺-Influx in die Erythrocyten via Na⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethyl-aminomethan) bei pH 7,4 und 37°C inkubiert. Die Erythrocyten wurden danach dreimal mit eiskalter M_{g}Cl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amiloridhemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

### Inhibition des Na⁺/H⁺-Exchangers:

| Beispiel | IC₅₀(mol / l) |
|---|---|
| 1: | 3.4 x 10⁻⁶ |
| 2: | 3.2 x 10⁻⁶ |
| 3: | 4.1 x 10⁻⁶ |

## Patentansprüche

1. Verwendung einer Verbindung der Formel I 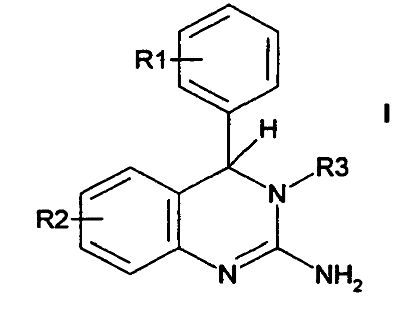 sowie von deren pharmazeutisch verträglichen Salzen, in welcher Formel I bedeuten:
R1 und R2 Wasserstoff, F, Cl, Br, I, geradkettiges oder verzweigtes C₁-C₄- Alkyl, C₁₋C₄- Alkoxy,
R3 Wasserstoff, geradkettiges oder verzweigtes C₁-C₄- Alkyl oder Phenyl,
wobei der Phenylkem unsubstituiert ist oder einen bis drei Substituenten ausgewählt aus der Gruppe bestehend aus F, Cl, CH₃ und CH₃O trägt;
zur Herstellung eines Medikaments zur Therapie und Prophylaxe ischämischer Zustände.

2. Verwendung nach Anspruch 1, **dadurch gekennzeichnet, dass** in Formel 1 bedeuten:
R1 und R2 Wasserstoff, Fluor, Chlor, geradkettiges oder verzweigtes C₁-C₄- Alkyl, C₁₋C₄- Alkoxy;
R3 Wasserstoff oder Methyl.

3. Verwendung nach Ansprüchen 1 bis 2 einer Verbindung I zur Herstellung eines Medikaments zur Behandlung oder Prophylaxe des Herzinfarkts.

4. Verwendung nach Ansprüchen 1 bis 2 einer Verbindung I zur Herstellung eines Medikaments zur Behandlung oder Prophylaxe der Angina Pectoris.

5. Verwendung nach Ansprüchen 1 bis 2 einer Verbindung I zur Herstellung eines Medikaments zur Behandlung oder Prophylaxe von ischämischen Zuständen des Herzens.

6. Verwendung nach Ansprüchen 1 bis 2 einer Verbindung I zur Herstellung eines Medikaments zur Behandlung oder Prophylaxe von ischämischen Zuständen des peripheren und zentralen Nervensystems und des Schlaganfalls.

7. Verwendung nach Ansprüchen 1 bis 2 einer Verbindung I zur Herstellung eines Medikaments zur Behandlung oder Prophylaxe von ischämischen Zuständen peripherer Organe und Gliedmaßen.

8. Verwendung nach Ansprüchen 1 bis 2 einer Verbindung I zur Herstellung eines Medikaments zur Behandlung von Schockzuständen.

## Revendications

1. Utilisation d'un composé de formule I 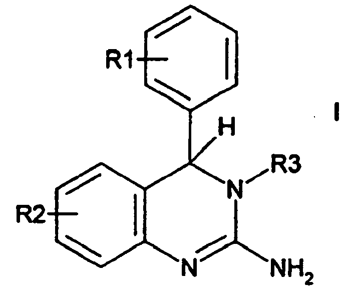 ainsi que de ses sels pharmaceutiquement acceptables, où, dans la formule I :
R1 et R2 signifient hydrogène, F, Cl, Br, I, alkyle en C₁ à C₄ linéaire ou ramifié, alcoxy en C₁ à C₄,
R3 signifie hydrogène, alkyle en C₁ à C₄ linéaire ou ramifié ou phényle,
le noyau phényle étant non substitué ou portant un à trois substituants choisis dans le groupe constitué par F, Cl, CH₃ et CH₃O ;
pour la préparation d'un médicament destiné à la thérapie et à la prophylaxie d'états ischémiques.

2. Utilisation selon la revendication 1, **caractérisée en ce que**, dans la formule I,
R1 et R2 signifient hydrogène, fluor, chlore, alkyle en C₁ à C₄ linéaire ou ramifié, alcoxy en C₁ à C₄;
R3 signifie hydrogène ou méthyle.

3. Utilisation selon les revendications 1 à 2 d'un composé I pour la préparation d'un médicament destiné au traitement ou à la prophylaxie de l'infarctus cardiaque.

4. Utilisation selon les revendications 1 à 2 d'un composé I pour la préparation d'un médicament destiné au traitement ou à la prophylaxie de l'angine de poitrine.

5. Utilisation selon les revendications 1 à 2 d'un composé I pour la préparation d'un médicament destiné au traitement ou à la prophylaxie d'états ischémiques du cerveau.

6. Utilisation selon les revendications 1 à 2 d'un composé I pour la préparation d'un médicament destiné au traitement ou à la prophylaxie d'états ischémiques du système nerveux central et périphérique et de l'attaque.

7. Utilisation selon les revendications 1 à 2 d'un composé I pour la préparation d'un médicament destiné au traitement ou à la prophylaxie d'états ischémiques d'organes périphériques et des membres.

8. Utilisation selon les revendications 1 à 2 d'un composé I pour la préparation d'un médicament pour le traitement d'états de choc.

## Claims

1. The use of a compound of the formula I 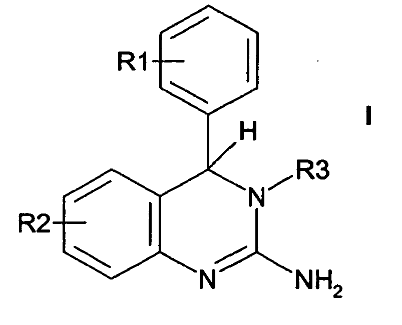 or of its pharmaceutically tolerable salts, in which formula I:
R1 and R2 are hydrogen, F, Cl, Br, I, straight-chain or branched C₁-C₄-alkyl, C₁-C₄-alkoxy,
R3 is hydrogen, straight-chain or branched C₁-C₄-alkyl or phenyl,
the phenyl nucleus being unsubstituted or carrying one to three substituents selected from the group consisting of F, Cl, CH₃ and CH₃O;
for the production of a medicament for the therapy and prophylaxis of ischemic conditions.

2. The use as claimed in claim 1, wherein in formula I:
R1 and R2 are hydrogen, fluorine, chlorine, straight-chain or branched C₁-C₄₋alkyl, C₁-C₄-alkoxy;
R3 is hydrogen or methyl.

3. The use as claimed in claims 1 to 2 of a compound I for the production of a medicament for the treatment or prophylaxis of cardiac infarct.

4. The use as claimed in claims 1 to 2 of a compound I for the production of a medicament for the treatment or prophylaxis of angina pectoris.

5. The use as claimed in claims 1 to 2 of a compound I for the production of a medicament for the treatment or prophylaxis of ischemic conditions of the heart.

6. The use as claimed in claims 1 to 2 of a compound I for the production of a medicament for the treatment or prophylaxis of ischemic conditions of the peripheral and central nervous system and of stroke.

7. The use as claimed in claims 1 to 2 of a compound I for the production of a medicament for the treatment or prophylaxis of ischemic conditions of peripheral organs and limbs.

8. The use as claimed in claims 1 to 2 of a compound I for the production of a medicament for the treatment of states of shock.
